# FASCICULE DE BREVET EUROPEEN

(11) **EP 1 670 700 B1**
(45) Date de publication et mention de la délivrance du brevet: **28.03.2007**
(21) Numéro de dépôt: 04805733.5
(22) Date de dépôt: 06.10.2004
(51) Int. Cl.: B65D 83/16

(54) **TÊTE DE DISTRIBUTION DE PRODUIT FLUIDE.**
FLUIDPRODUKTABGABEKOPF
FLUID PRODUCT DISPENSING HEAD

(30) Priorité: 09.10.2003 FR 0311822
(43) Date de publication de la demande: 21.06.2006
(73) Titulaire: Airlessystems, 27380 Charleval (FR)
(72) Inventeur: DECOTTIGNIES, Laurent, F-95800 Cergy (FR); BEHAR, Alain, F-92510 Suresnes (FR)
(74) Mandataire: CAPRI
(86) Numéro de dépôt international: PCT/FR2004/050490
(87) Numéro de publication internationale: WO 2005/032973

(56) Documents cités:
- DE-A- 4 002 817
- DE-U- 7 901 179
- FR-A- 2 453 790
- US-A- 3 096 002
- US-A- 3 317 092

## Description

La présente invention concerne une tête de distribution de produit fluide destinée à être montée sur ou associée à une tige d'actionnement d'un organe de distribution déplaçable en va-et-vient selon un axe longitudinal de déplacement. La tige définit intérieurement un canal de refoulement à travers lequel le produit fluide est acheminée à la tête de distribution. L'organe de distribution peut se présenter sous la forme d'une pompe ou d'une valve comprenant un corps à l'intérieur duquel la tige d'actionnement est montée de manière déplaçable en va-et-vient à l'encontre d'un ressort de rappel qui tend à renvoyer la tige d'actionnement dans une position de repos. La tête de distribution est ainsi associée à une pompe ou une valve pour constituer un distributeur de produit fluide également pourvu d'un réservoir de produit fluide à partir duquel la pompe ou la valve prélève du produit fluide pour être distribué à travers la tête. De telles têtes de distribution sont fréquemment utilisées dans les domaines de la parfumerie, de la cosmétique ou encore de la pharmacie.

En général, de telles têtes de distribution comprennent un manchon de raccordement axial destiné à être engagé sur l'extrémité libre de la tige d'actionnement. D'autre part, la tête comprend également un embout de distribution définissant un canal d'embout relié au conduit d'entrée par un canal de liaison. L'embout comprend une extrémité libre de distribution définissant un orifice de distribution situé à une extrémité aval du canal d'embout. La tête comprend également une surface d'appui sur laquelle une pression axiale peut être exercée pour enfoncer la tige d'actionnement. La tête de distribution remplit donc une fonction de poussoir et de distribution à la fois. Ce genre de tête de distribution est déjà connu de l'art antérieur. Elle comporte un embout de distribution incliné par rapport à l'axe longitudinal de déplacement. Du fait de l'inclinaison de l'embout de distribution, la tête de distribution est difficile à fabriquer : en effet, étant donné que cette tête est réalisée par moulage, l'inclinaison de l'embout nécessite un moule relativement compliqué qui n'est pas déplaçable selon un axe unique.

Le document DE 4002817 A décrit une telle tête de distribution, conformément au préambule de la revendication 1.

La présente invention a pour but de simplifier la fabrication d'une telle tête de distribution. Un autre but de l'invention est de pourvoir la tête de distribution d'une fonction d'applicateur de produit fluide sur une surface d'application. Un autre but est de garantir la conservation du produit fluide à l'intérieur de la tête, même à proximité de l'orifice de distribution. Un autre but encore est de simplifier le montage d'une telle tête de distribution. Encore un autre but est de réaliser cette tête de distribution avec un nombre minimum de pièces constitutives.

Pour atteindre ces buts, la présente invention propose une tête de distribution de produit fluide selon la revendication 1 l'embout s'étend donc sensiblement parallèlement de manière décalée par rapport à l'axe longitudinal de déplacement de la tige d'actionnement. Le fait que l'embout s'étende parallèlement à l'axe facilite aussi bien le moulage que le montage de la tête de distribution. Avantageusement, la surface d'appui peut s'étendre axialement en aval du manchon de raccordement en coupant ledit axe. En décalant l'embout, cela permet de dégager une surface d'appui relativement étendue qui permet l'application d'une force de pression parfaitement centrée, puisque située sur l'axe longitudinal d'actionnement.

Selon un autre aspect intéressant de l'invention, la tête, de distribution peut comprendre une couronne de base qui s'étend autour du manchon de raccordement, l'embout étant inscrit axialement dans cette couronne. Avantageusement, l'embout peut être axialement tangent à ladite couronne. Cela signifie que la couronne définit l'encombrement extérieur radial de la tête de distribution. En d'autres termes, cela signifie également que l'embout de distribution ne s'étend pas radialement au-delà du pourtour de la couronne.

Selon une autre caractéristique intéressante de l'invention, la tête de distribution peut comprendre un noyau interne sensiblement rigide et une enveloppe externe sensiblement souple, ledit noyau étant en prise dans ladite enveloppe. Avantageusement, le noyau peut être reçu axialement dans l'enveloppe. Il est à noter que cette caractéristique de conception de la tête de distribution en un noyau et une enveloppe peut être mise en oeuvre indépendamment du fait que l'embout s'étende parallèlement de manière décalée par rapport à l'axe. Le noyau sensiblement rigide constitue une structure solide destinée à venir en prise avec la tige d'actionnement. Quant à l'enveloppe souple, elle permet de réaliser des fonctions nécessitant une déformabilité de la matière.

Selon un mode de réalisation avantageux, le noyau forme le manchon de raccordement, partiellement le canal de liaison et avantageusement partiellement une partie basse du canal d'embout. D'autre part, l'enveloppe peut former l'embout de distribution et une paroi d'appui définissant la surface d'appui. Avantageusement, le noyau forme une broche axiale engagée dans l'embout, une partie basse du canal d'embout étant formée entre l'enveloppe et la broche. Avantageusement, la broche comprend une saignée axiale formant la partie basse du canal d'embout avec l'embout de distribution. De préférence, la broche comprend une extrémité supérieure qui se termine en éloignement de l'orifice de distribution, une partie haute du canal d'embout étant uniquement formé par l'enveloppe souple en aval de la broche, de sorte que l'embout est souple au niveau de la partie haute.

Selon un autre aspect de l'invention, le noyau forme un plateau d'appui au niveau duquel le conduit débouche axialement, le canal de liaison étant formé entre le plateau et l'enveloppe. Avantageusement, le plateau comprend une saignée transversale et non-axiale formant le canal de liaison avec l'enveloppe.

Selon un autre aspect de l'invention, le noyau forme un collier en prise dans une couronne de base formée par l'enveloppe. Là encore, le noyau permet de conférer une certaine tenue à l'enveloppe souple.

Selon une autre caractéristique de l'invention qui peut être mise en oeuvre indépendamment des autres, l'embout de distribution peut présenter une forme plate de spatule. Ainsi, l'embout de distribution remplit une double fonction, à savoir celle de distribution et d'application de produit fluide à la fois. En effet, la forme plate de spatule permet un étalement aisé du produit fluide sur une surface d'application quelconque.

Selon une autre caractéristique intéressante, l'orifice de distribution peut être formé par une fente souple auto-jointante. Ceci est rendu possible du fait que l'enveloppe est réalisée en un matériau souple élastiquement déformable. La fente souple auto-jointante définit ainsi deux lèvres destinées à venir en contact étanche l'une de l'autre lorsque aucune pression n'est présente dans l'embout de distribution. On réalise ainsi un orifice de distribution de manière monobloc avec le restant de l'enveloppe souple, alors que dans l'art antérieur, l'orifice de distribution est souvent réalisé par un gicleur rapporté sur un autre élément de la tête.

Selon une forme de réalisation pratique, la surface d'appui peut être inclinée en faisant un angle allant de 40 à 90 degrés par rapport à l'axe de manière à couper cet axe. L'inclinaison de la surface d'appui permet détendre la superficie de la surface et de rendre en même temps la surface plus ergonomique.

Selon une autre caractéristique qui peut être mise en oeuvre indépendamment des autres caractéristiques, l'embout de distribution peut être au moins partiellement souple, notamment au niveau de son extrémité libre. En effet, on peut très bien imaginer une tête de distribution sans embout axial décalé mais définissant une partie d'extrémité souple formant également l'orifice de distribution. La tête peut ainsi comprendre un embout partiellement souple définissant une fente auto-jointante de distribution, qui permet, après distribution du produit fluide, d'appliquer ou d'étaler le produit fluide à l'aide de la partie souple de l'embout.

L'invention sera maintenant plus amplement décrite en référence aux dessins joints donnant à titre d'exemple non limitatif un mode de réalisation de l'invention. Sur les figures :
- la figure 1 est une vue en perspective éclatée selon l'axe X d'une tête de distribution selon un mode de réalisation de l'invention,
- la figure 2 est une vue en coupe transversale verticale à travers un distributeur de produit fluide équipé de la tête de distribution de la figure 1, et
- la figure 3 est une vue agrandie de la partie supérieure du distributeur de la figure 2 intégrant la tête de distribution selon l'invention.

La forme de réalisation utilisée pour donner un exemple de réalisation d'une tête de distribution de produit fluide selon l'invention est une tête du type poussoir sur laquelle on appuie pour commander la distribution du produit fluide. La tête de distribution est montée ou associée à un organe de distribution qui peut être une pompe ou une valve. Sur les dessins, l'organe de distribution est désigné par la référence numérique 3. La tête de distribution est désignée par les références numériques 1 et 2, puisque constituée de deux éléments distincts, à savoir un noyau 1 et une enveloppe 2.

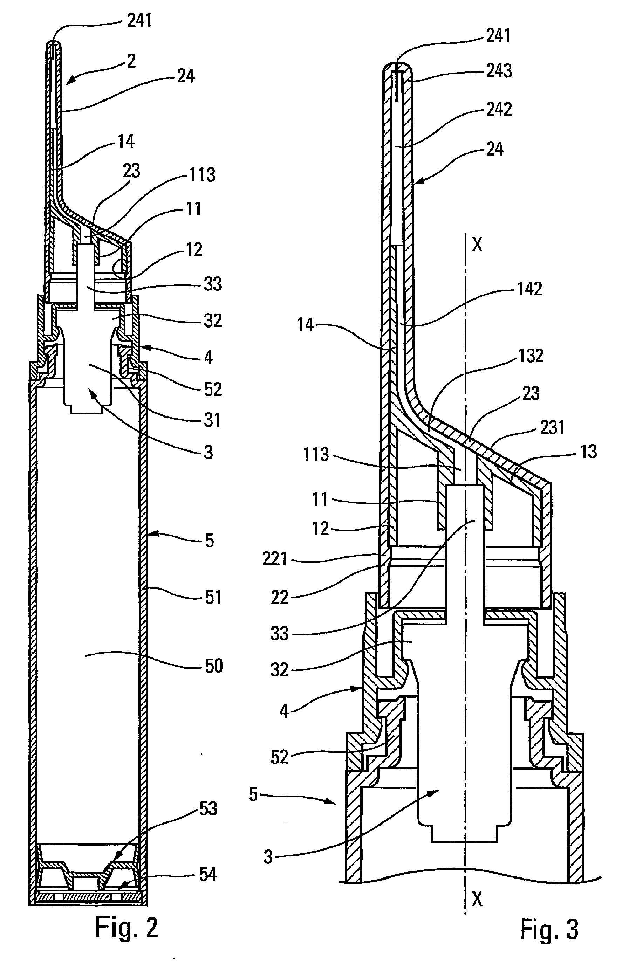

L'organe de distribution 3 comprend un corps 31 formant une collerette 32 en prise dans une bague de fixation 4. L'organe de distribution 3 comprend également une tige d'actionnement 33 montée de manière déplaçable en va-et-vient à l'intérieur du corps 31. La tige d'actionnement 33 est sollicitée en position de repos par un ressort de rappel (non représenté). Sur les figures 2 et 3, la tige d'actionnement est dans sa position de repos. La tige d'actionnement définit un canal de refoulement interne à travers lequel le produit fluide mis sous pression dans le corps de pompe 31 est refoulé pour être distribué à travers la tête de distribution. La tige s'étend et se déplace selon un axe longitudinal d'actionnement X qui peut être confondu avec les axes de symétrie du récipient, de la bague et du corps de l'organe de distribution.

La bague de fixation 4 qui est en prise avec le corps 31 de l'organe de distribution 3 est fixée sur le col 52 d'un récipient 5 définissant un réservoir interne 50. Le récipient 5 comprend un fût sensiblement cylindrique 51 à l'intérieur duquel peut coulisser un piston-suiveur ou racleur 53. Optionnellement, un fond rapporté 54 peut obturer l'extrémité inférieure du fût 51. Il s'agit là d'un récipient particulier dans lequel le volume interne du réservoir 50 diminue à mesure que du produit est distribué par l'organe de distribution. Toutefois, d'autres types de récipients, à contenance fixe ou variable, peuvent être utilisés dans le cadre de l'invention.

Le type d'organe de distribution, de bague de fixation et de récipient n'est pas critique pour la présente invention. Il suffit que l'organe de distribution soit pourvu d'une tige d'actionnement déplaçable en va-et-vient et définissant un conduit de refoulement interne.

La tête de distribution dans ce mode particulier non limitatif de l'invention comprend donc un noyau 1 et une enveloppe 2. Le noyau 1 est réalisé dans une matière sensiblement rigide, alors que l'enveloppe 2 est réalisée dans une matière relativement souple et élastiquement déformable. Le noyau et l'enveloppe peuvent être réalisés dans des matières plastiques conventionnelles, comme du polyéthylène, du polypropylène et des thermoplastiques élastomères. Le fait de réaliser la tête de distribution à partir d'un noyau et d'une enveloppe ne doit pas être considéré comme limitatif. En effet, une tête de distribution selon l'invention peut également être réalisée en une seule pièce monobloc, ou encore en plus de deux pièces. Cependant, dans le mode de réalisation représenté sur les dessins, la tête comprend un noyau et une enveloppe.

Le noyau 1 est de préférence réalisé de manière monobloc par injection de matière plastique. Le noyau 1 comprend un collier sensiblement cylindrique 12 dont la section est de préférence circulaire. Le collier 12 est obturé à son extrémité supérieure par un plateau d'appui 13 qui s'étend de manière inclinée par rapport à l'axe du cylindre formé par le collier 12. D'ailleurs, l'axe du cylindre 12 est confondu avec l'axe longitudinal d'actionnement X de la tige d'actionnement 33. Dans sa partie la plus élevée, le plateau d'appui 13 est pourvu d'une broche 14 qui se projette axialement vers le haut selon l'axe X, mais de manière décalée par rapport à cet axe. En effet, la broche 14 s'étend dans le prolongement de la paroi externe du collier 12, comme on peut le vois clairement sur la figure 3. La broche présente une extrémité supérieure 141. D'autre part, la broche, sur sa face tournée vers l'axe X est formée avec une saignée axiale 142' dont l'extrémité inférieure se raccorde à une autre saignée transversale ou non-axiale 132' qui est formée dans l'épaisseur du plateau d'appui 13. Les deux saignées 132' et 142', s'étendent donc dans le prolongement l'une de l'autre en faisant un angle au niveau de la base de la broche 14 qui se raccorde au plateau 13. L'extrémité inférieure de la saignée non-axiale 132' débouche dans un conduit 113 qui traverse l'épaisseur du plateau 13. Le plateau 13 définit ainsi une surface supérieure 131 qui est relativement ou parfaitement plane, à l'exception de l'endroit où sont formés la saignée non-axiale 132' et le conduit 113. Le noyau 1 forme également un manchon de raccordement 11 qui forme intérieurement le conduit d'entrée 113. Le manchon 11 est de forme générale cylindrique avec un axe de cylindre qui est confondu avec l'axe X de la tige d'actionnement une fois la tête de distribution montée sur la tige d'actionnement.

L'enveloppe 2 est de préférence réalisée de manière monobloc à partir d'une matière plastique moulée, comme du thermoplastique élastomère. L'enveloppe 2 comprend une couronne 22 qui est sensiblement cylindrique circulaire. La couronne 22 s'étend également selon l'axe X, une fois montée sur la tige. La couronne 22 est obturée à son extrémité supérieure par une paroi d'appui 23 qui forme une surface d'appui externe 231. La paroi 23 s'étend de manière inclinée par rapport à l'axe X. L'angle d'inclinaison de la paroi 23 par rapport à l'axe X peut être de l'ordre de 40 à 90° par rapport à l'axe X, c'est-à-dire par rapport à la verticale. En d'autres termes, la paroi d'appui 23 peut être horizontale ou encore relativement pentue. Bien évidemment, plus la pente est importante, plus la superficie de la paroi 23 augmente. La surface d'appui 231 est destinée à l'application d'une force d'appui à l'aide d'un ou de plusieurs doigt(s) de la main. Plus la surface est étendue, plus il est aisé d'apposer un ou plusieurs doigt(s). Il faut cependant remarquer que la surface d'appui 231 est située de manière centrée sur l'axe X. En effet, la paroi d'appui 23 est située axialement en aval du conduit d'entrée axial 113. En d'autres termes, la paroi d'appui 23 coupe l'axe X. La force d'appui exercée par le doigt de l'utilisateur va donc être appliquée directement selon l'axe X, ce qui permet de garantir une bonne répartition de la force d'appui sur la tête lors de la distribution. La paroi d'appui 23, bien que réalisée en un matériau souple, ne peut pas se déformer, étant donné qu'elle est contact du plateau d'appui 13 formé par le noyau rigide 1. La paroi d'appui 23 recouvre également le conduit 113 et la saignée non-axiale 132' sans les boucher. Au contraire, la surface inférieure de la paroi d'appui 23 vient compléter la saignée non axiale 132' pour former un canal de liaison 132 visible sur la figure 3. De même, la surface inférieure de la paroi d'appui 23 forme une paroi de déviation à la sortie du conduit d'entrée axial 113 de manière à pouvoir dévier le produit fluide du conduit 113 dans le canal de liaison 132. Il faut également noter que le canal de liaison 132 est relativement étanche, bien que la paroi d'appui 23 ne soit pas fixée ou soudée sur le plateau 13. En effet, lors de la distribution, l'utilisateur va exercer une pression sur la surface d'appui 131 ce qui a tendance à plaquer fortement la paroi 23 sur le plateau 13. Ainsi, la sortie du conduit 13 ainsi que le canal de liaison 132 sont parfaitement isolés. La matière relativement souple de la paroi d'appui 23 contribue également à isoler le canal de liaison 132.

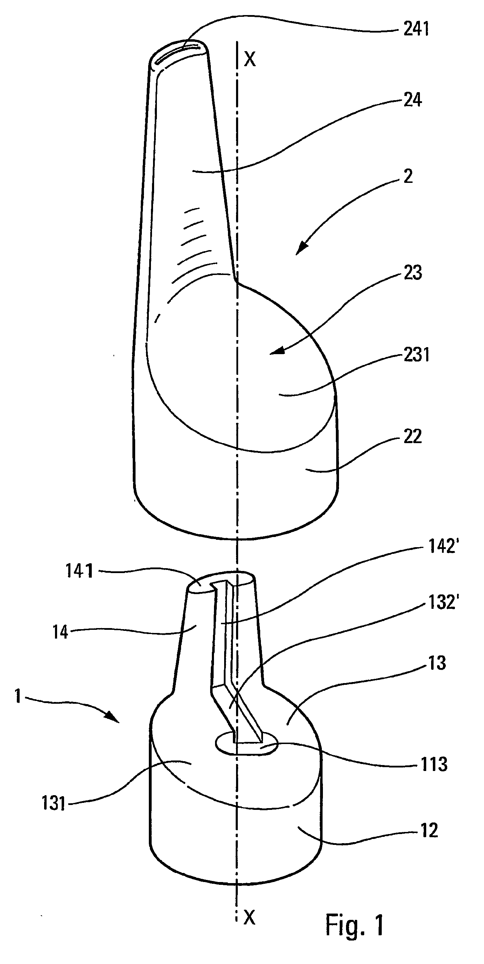

Au niveau de son point le plus haut, la paroi d'appui 23 forme un embout de distribution 24 qui se projette axialement vers le haut de manière décalée par rapport à l'axe X. L'embout de distribution 24 s'étend de manière décalée et axiale, c'est-à-dire parallèlement le long à l'axe X. On peut notamment remarquer sur les figures 2 et 3 que l'embout de distribution 24 s'étend de manière désaxée dans le prolongement de la tangente ou du bord de la couronne 22 : la paroi externe de l'embout 24 est parfaitement alignée avec la paroi externe de la couronne 22 sur la partie gauche comme on peut le voir sur les figures 2 et 3. On peut également dire que l'embout 24 est inscrit axialement dans la couronne 22. En d'autres termes, l'embout 24 ne fait pas saillie radialement vers l'extérieur par rapport à la couronne 22, lorsque l'on regarde l'enveloppe de dessus ou de dessous selon l'axe X. Il s'agit là d'une caractéristique qui peut être protégée indépendamment du fait que la tête de distribution est réalisée en deux pièces. On peut notamment imaginer une tête de distribution formant une surface d'appui et un embout de distribution axial décalé qui s'étend ou est inscrit dans la couronne de base de la tête de distribution. Le fait que l'embout s'étende de manière axiale permet un moulage un démoulage et un montage aisés de la pièce. En effet, à l'aide d'une seule broche, on peut mouler l'intérieur de la tête de distribution. De plus, elle peut être réalisée de manière monobloc. Dans le cas où la tête est réalisée en deux pièces, c'est-à-dire avec un noyau 1 et une enveloppe 2 comme c'est le cas dans le mode de réalisation représenté sur les figures, l'orientation axiale de l'embout inscrite dans la couronne permet également un montage aisé et simple du noyau 1 à l'intérieur de l'enveloppe 2. Ce montage peut être réalisé en engageant le noyau 1 dans l'enveloppe 2 de manière parfaitement axiale. Le collier rigide 12 vient en prise à l'intérieur de la couronne 22 alors que le plateau 13 vient en contact de la paroi d'appui 23. D'autre part, la broche 14 pénètre à l'intérieur de l'embout 24. Comme précédemment mentionné, la paroi d'appui 23 vient compléter le canal de liaison 132 formé par la saignée 132' réalisée dans la plateau 13. D'autre part, l'embout 24 vient compléter la saignée axiale 142' formée dans la broche 14 de manière à réaliser une partie basse 142 d'un canal d'embout qui s'étend à l'intérieur de l'embout 24. Cette partie basse 142 du canal d'embout se termine au niveau de l'extrémité supérieure 141 de la broche 14. Au-delà de cette extrémité 141, l'embout 24 forme une partie haute 242 du canal d'embout qui se prolonge jusqu'à une extrémité aval formant un orifice de distribution 141, sous la forme d'une fente auto-jointante. Ainsi, toute la partie d'extrémité libre 243 de l'embout 24 formée uniquement par l'embout 24 présente une caractéristique de déformabilité élastique à la manière d'une spatule souple. D'ailleurs, l'embout 24 présente une forme plate de spatule comme on peut le voir sur la figure 1. En effet, l'embout 24 est plus large tangentiellement que radialement. Ceci est très visible en comparant la figure 1 avec la figure 3. Le fait de réaliser un embout de distribution au moins partiellement souple avec un orifice de distribution, par exemple sous la forme d'une fente auto-jointante, est une caractéristique qui peut être mise en oeuvre indépendamment du fait que l'embout s'étend de manière axialement décalée ou encore que la tête est réalisée à partir d'un noyau et d'une enveloppe. On peut très bien imaginer une tête de distribution formant un embout axial transversal ou horizontal dont la partie d'extrémité libre est souple et forme un orifice de distribution rapporté ou monobloc. Dans le cas où l'orifice de distribution est réalisé sous la forme d'une fente auto-jointante 241, cette fente peut aisément être réalisée en incisant l'extrémité de l'embout 24 à l'aide d'une lame. On réalise ainsi une fente constituée de deux lèvres qui viennent en contact étanche mutuel lorsque aucune pression ne règne à l'intérieur du canal d'embout. Ce type d'orifice de distribution réalise en même temps une fonction d'obturation permettant de conserver le produit fluide à l'abri de l'air extérieur à l'intérieur de l'embout 24.

A l'inverse, on peut également imaginer que la broche 14 s'étende jusqu'au niveau de l'orifice de distribution 241. Dans ce cas, l'embout 24 ne présente une caractéristique de déformabilité élastique plus réduite, voire néante.

Le fait de réaliser l'embout 24 sous la forme d'une spatule plate, avantageusement élastiquement déformable, permet d'utiliser l'embout en tant qu'applicateur de produit fluide pour appliquer et/ou étaler le produit fluide sur une surface d'application.

En outre, l'orientation parfaitement ou sensiblement axiale décalée de l'embout 24 avec avantageusement une surface d'appui parfaitement centrée sur l'axe permet d'obtenir une bonne précision dans la localisation de la distribution et de l'application du produit fluide sur une surface d'application.

Le montage d'une telle tête de distribution constituée à partir d'un noyau 1 et d'une enveloppe 2 est très simple, puisqu'il suffit d'insérer axialement le noyau rigide 1 dans l'enveloppe souple 2. Ceci est possible du fait que la broche 14 ainsi que l'embout 24 s'étendent de manière axiale. La broche 14 pénètre dans l'embout 24 pour former la partie basse 142 du canal d'embout, le plateau 13 vient en contact de la paroi 23 pour former le canal de liaison 132 et le collier 12 vient en prise dans la couronne 22. On peut même prévoir un profil d'encliquetage 221 au niveau de la paroi interne de la couronne 22 pour garantir un maintien stable et définitif du collier 12 dans l'enveloppe 2.

Diverses modifications sont possibles sans pour autant sortir du cadre de l'invention. La broche 14 peut être plus ou moins longue. Il en est de même pour l'embout 24. La broche 14 peut former uniquement le canal de liaison 132 sans former une partie du canal d'embout. La broche 14 peut également former l'intégralité du canal d'embout. On peut par exemple imaginer que la partie basse 142 du canal d'embout soit entièrement formée à l'intérieur de la broche 14. On peut aussi imaginer d'autres types d'orifices de distribution que celui sous la forme d'une fente auto-jointante. Un orifice de distribution constamment ouvert est envisageable. Une surface d'appui parfaitement horizontale est également envisageable. Une fabrication de la tête de distribution de manière monobloc ou en bi-injection est également envisageable. On peut notamment envisager de surmouler l'enveloppe sur le noyau, en prévoyant certains aménagements conventionnels.

Il est également avantageux que l'extrémité inférieure de la couronne 22 s'étende à l'intérieur d'une douille formée par la bague de fixation 4. Ainsi, la tige d'actionnement 33 n'est pas visible.

## Revendications

1. - Tête de distribution de produit fluide destinée à être montée sur une tige d'actionnement (33) d'un organe de distribution (3) déplaçable en va-et-vient selon un axe (X), ladite tête comprenant un manchon de raccordement axial (11) destiné à être engagé sur la tige d'actionnement (33) et définissant un conduit d'entrée (113), ladite tête comprenant en outre un embout de distribution (24) définissant un canal d'embout (142, 242) relié au conduit d'entrée (113) par un canal de liaison (132), ledit embout (21) comprenant un orifice de distribution (241) situé à une extrémité aval du canal d'embout, ladite tête comprenant une surface d'appui (231) sur laquelle une pression axiale peut être exercée pour enfoncer la tige d'actionnement (33), l'embout (24) s'étendant sensiblement parallèlement de manière décalée par rapport audit axe (X), **caractérisée en ce que** la surface d'appui (231) s'étend axialement en aval du manchon de raccordement (11) en coupant ledit axe (X).

2. - Tête de distribution selon la revendication 1, comprenant une couronne de base (22) qui s'étend autour du manchon de raccordement (11), l'embout (24) étant inscrit axialement dans cette couronne.

3. - Tête de distribution selon la revendication 2, dans laquelle l'embout (24) est axialement tangent à ladite couronne (22).

4. - Tête de distribution selon l'une quelconque des revendications précédentes, comprenant un noyau interne sensiblement rigide (1) et une enveloppe externe sensiblement souple (2), ledit noyau étant en prise dans ladite enveloppe.

5. - Tête de distribution selon la revendication 4, dans laquelle le noyau (1) est reçu axialement dans l'enveloppe (2).

6. - Tête de distribution selon la revendication 4 ou 5, dans laquelle le noyau (1) forme le manchon de raccordement (11), partiellement le canal de liaison (132) et avantageusement partiellement une partie basse (142) du canal d'embout.

7. - Tête de distribution selon la revendication 4, 5 ou 6, dans laquelle l'enveloppe (2) forme l'embout de distribution (24) et une paroi d'appui (23) définissant la surface d'appui (231).

8. - Tête de distribution selon la revendication 7, dans laquelle le noyau (1) forme une broche axiale (14) engagée dans l'embout (24), une partie basse (142) du canal d'embout étant formée entre l'enveloppe (2) et la broche (14).

9. - Tête de distribution selon la revendication 8, dans laquelle la broche (14) comprend une saignée (142') axiale formant la partie basse (142) du canal d'embout avec l'embout de distribution (24).

10. - Tête de distribution selon la revendication 8 ou 9, dans laquelle la broche (14) comprend une extrémité (141) qui se termine en éloignement de l'orifice de distribution (241), une partie haute (242) du canal d'embout étant uniquement formé par l'enveloppe souple (2) en aval de la broche, de sorte que l'embout est souple au niveau de la partie haute, l'orifice de distribution (241) étant formé au niveau de la partie haute.

11. - Tête de distribution selon l'une quelconque des revendications 4 à 10, dans laquelle le noyau (1) forme un plateau d'appui (13) au niveau duquel le conduit (113) débouche axialement, le canal de liaison (132) étant formé entre le plateau (13) et l'enveloppe (2).

12. - Tête de distribution selon la revendication 11, dans laquelle le plateau (13) comprend une saignée transversale (132') formant le canal de liaison (132) avec l'enveloppe (2).

13. - Tête de distribution selon l'une quelconque des revendications 4 à 12, dans laquelle le noyau (1) forme un collier (12) en prise dans une couronne de base (22) formée par l'enveloppe (2).

14. - Tête de distribution selon l'une quelconque des revendications précédentes, dans laquelle l'embout de distribution (24) présente une forme plate de spatule.

15. - Tête de distribution selon l'une quelconque des revendications précédentes, dans laquelle l'orifice de distribution est formé par une fente souple auto-jointante (241).

16. - Tête de distribution selon l'une quelconque des revendications précédentes, dans laquelle la surface d'appui (231) est inclinée en faisant un angle allant de 40 à 90 degrés par rapport à l'axe de manière à couper cet axe.

17. - Tête de distribution selon l'une quelconque des revendications précédentes, dans laquelle l'embout de distribution (24) est au moins partiellement souple, notamment au niveau de son extrémité libre (243).

## Claims

1. A fluid dispenser head for mounting on an actuator rod (33) of a dispenser member (3) that is displaceable down and up along an axis (X), said head comprising an axial connection sleeve (11) for engaging on the actuator rod (33) and defining an inlet duct (113), said head further comprising a dispenser endpiece (24) defining an endpiece channel (142, 242) that is connected to the inlet duct (113) via a connection channel (132), said endpiece (24) including a dispenser orifice (241) that is situated at a downstream end of the endpiece channel, said head further comprising a bearing surface (231) on which axial pressure can be exerted so as to drive in the actuator rod (33), the endpiece (24) extending substantially parallel to said axis (X), and being offset away from the axis, the dispenser head being **characterized in that** the bearing surface (231) extends axially downstream from the connection sleeve (11), intersecting said axis (X).

2. A dispenser head according to claim 1, further comprising a base skirt (22) that extends around the connection sleeve (11), the endpiece (24) being inscribed within the outline of the skirt.

3. A dispenser head according to claim 2, in which the endpiece (24) is axially tangential to said skirt (22).

4. A dispenser head according to any preceding claim, comprising a substantially rigid inner core (1), and a substantially flexible outer casing (2), said core being engaged in said casing.

5. A dispenser head according to claim 4, in which the core (1) is received axially in the casing (2).

6. A dispenser head according to claim 4 or claim 5, in which the core (1) forms the connection sleeve (11), part of the connection channel (132), and advantageously part of a bottom portion (142) of the endpiece channel.

7. A dispenser head according to claim 4, 5, or 6, in which in which the casing (2) forms the dispenser endpiece (24), and a bearing wall (23) defining the bearing surface (231).

8. A dispenser head according to claim 7, in which the core (1) forms an axial spout (14) that is engaged in the endpiece (24), a bottom portion (142) of the endpiece channel being formed between the casing (2) and the spout (14) .

9. A dispenser head according to claim 8, in which the spout (14) includes an axial groove (142') that cooperates with the dispenser endpiece (24) to form the bottom portion (142) of the endpiece channel. '

10. A dispenser head according to claim 8 or claim 9, in which the spout (14) includes an end (141) that terminates in a position set back from the dispenser orifice (241), a top portion (242) of the endpiece channel being formed solely by the flexible casing (2) downstream from the spout, such that the endpiece is flexible at the top portion, the dispenser orifice (241) being formed in the top portion.

11. A dispenser head according to any one of claims 4 to 10, in which the core (1) forms a bearing plate (13) into which the duct (113) opens out axially, the connection channel (132) being formed between the plate (13) and the casing (2).

12. A dispenser head according to claim 11, in which the plate (13) includes a transverse groove (132') that cooperates with the casing (2) to form the connection channel (132).

13. A dispenser head according to any one of claims 4 to 12, in which the core (1) forms a collar (12) that is engaged in a base skirt (22) formed by the casing (2).

14. A dispenser head according to any preceding claim, in which the dispenser endpiece (24) presents a flat spatula shape.

15. A dispenser head according to any preceding claim, in which the dispenser orifice is formed by a self-sealing flexible slot (241).

16. A dispenser head according to any preceding claim, in which the bearing surface (231) slopes, forming an angle lying in the range 40° to 90° relative to the axis, in such a manner as to intersect the axis.

17. A dispenser head according to any preceding claim, in which the dispenser endpiece (24) is flexible, at least in part, in particular at its free end (243).

## Patentansprüche

1. Fluidprodukt-Abgabekopf zur Anbringung auf einer Betätigungsstange (33) eines Abgabeorgans (3), welches entlang einer Achse (X) hin- und herbewegbar ist, wobei der Kopf eine axiale Verbindungsmuffe (11) aufweist, die dazu bestimmt ist, auf der Betätigungsstange (33) in Eingriff zu gelangen und die eine Einlassleitung (113) festlegt, wobei der Kopf außerdem ein Abgabemundstück (24) aufweist, welches einen Mundstückkanal (142,242) festlegt, welcher mit der Einlassleitung (113) über einen Verbindungskanal (132) verbunden ist, wobei das Mundstück (21) eine Abgabeöffnung (241) aufweist, welche an einem stromabwärtigen Ende des Mundstückkanals angeordnet ist, wobei der Kopf eine Anlagefläche (231) aufweist, auf welche ein axialer Druck ausgeübt werden kann, um die Betätigungsstange (33) niederzudrücken, wobei das Mundstück (24) sich im wesentlichen parallel und versetzt zur Achse (X) erstreckt, **dadurch gekennzeichnet, dass** die Anlagefläche (231) sich axial stromabwärts von der Verbindungsmuffe (11) erstreckt und die Achse (X) schneidet.

2. Abgabekopf nach Anspruch 1, aufweisend eine Basiskrone (22), welche sich um die Verbindungsmuffe (11) erstreckt, wobei das Mundstück (24) sich axial in diese Krone absenkt.

3. Abgabekopf nach Anspruch 2, wobei das Mundstück (24) axial eine Tangente an die Krone (22) bildet.

4. Abgabekopf nach einem der vorangehenden Ansprüche, aufweisend einen im wesentlichen starren Innenkern (1) und eine im wesentlichen nachgiebige äußere Hülle (2), wobei der Kern in die Hülle eingreift.

5. Abgabekopf nach Anspruch 4, wobei der Kern (1) axial in der Hülle (2) aufgenommen ist.

6. Abgabekopf nach Anspruch 4 oder 5, wobei der Kern (1) die Verbindungsmuffe (11), teilweise den Verbindungskanal (132) und vorteilhafterweise teilweise einen unteren Teil (142) des Mundstückkanals bildet.

7. Abgabekopf nach Anspruch 4, 5 oder 6, wobei die Hülle (2) das Abgabemundstück (24) und eine Anlagewand (23) bildet, welche die Anlagefläche (231) festlegt.

8. Abgabekopf nach Anspruch 7, wobei der Kern (1) einen axialen Zapfen (14) bildet, der in das Mundstück (24) eingreift, wobei ein unterer Teil (142) des Mundstückkanals zwischen der Hülle (2) und dem Zapfen (14) gebildet ist.

9. Abgabekopf nach Anspruch 8, wobei der Zapfen (14) ein axiales Ablaufmittel (142') umfasst, welches den untern Teil (142) des Mundstückkanals mit dem Abgabemundstück (24) bildet.

10. Abgabekopf nach Anspruch 8 oder 9, wobei der Zapfen (14) ein Ende (141) aufweist, welches abgekehrt von der Abgabeöffnung (241) endet, wobei ein oberer Teil (242) des Mundstückkanals durch die nachgiebige Hülle (2) stromaufwärts von dem Zapfen gemeinsam gebildet ist, so dass das Mundstück auf der Höhe des oberen Teils nachgiebig ist, wobei die Mundstücköffnung (241) auf der Höhe des oberen Teils gebildet ist.

11. Abgabekopf nach einem der Ansprüche 4 bis 10, wobei der Kern (1) ein Anlageplateau (10) bildet, auf dessen Höhe der Kanal (113) axial ausmündet, wobei der Verbindungskanal (132) zwischen dem Plateau (13) und der Hülle (2) gebildet ist.

12. Abgabekopf nach Anspruch 1, wobei das Plateau (13) einen Querablauf (132') aufweist, der den Verbindungskanal (132) mit der Hülle (2) bildet.

13. Abgabekopf nach einem der Ansprüche 4 bis 12, wobei der Kern (1) einen Kragen (12) im Eingriff in eine Basiskrone (2) bildet, welche durch die Hülle (2) gebildet ist.

14. Abgabekopf nach einem der vorangehenden Ansprüche, wobei das Abgabemundstück (24) eine spatelförmige flache Form besitzt.

15. Abgabekopf nach einem der vorangehenden Ansprüche, wobei die Abgabeöffnung durch einen nachgiebigen, selbst verschlie-ßenden Schlitz gebildet ist.

16. Abgabekopf nach einem der vorangehenden Ansprüche, wobei die Anlagefläche (231) unter einen Winkel von 40 bis 90 Grad relativ zu der Achse derart geneigt ist, dass sie die Achse schneidet.

17. Abgabekopf nach einem der vorangehenden Ansprüche, wobei das Abgabemundstück (24) zumindest teilweise, insbesondere auf der Höhe seines freien Endes (243) nachgiebig gebildet ist.
